(19) Europäisches Patentamt

European Patent Office

Office européen des brevets

(11) **EP 1 076 823 B1**

(12) **EUROPÄISCHE PATENTSCHRIFT**

(45) Veröffentlichungstag und Bekanntmachung des Hinweises auf die Patenterteilung:
**04.12.2002 Patentblatt 2002/49**

(51) Int Cl.⁷: **G01N 33/543**, G01N 21/88, G02B 19/00

(21) Anmeldenummer: **99907618.5**

(22) Anmeldetag: **10.03.1999**

(86) Internationale Anmeldenummer:
**PCT/EP99/01548**

(87) Internationale Veröffentlichungsnummer:
**WO 99/046596 (16.09.1999 Gazette 1999/37)**

(54) **OPTISCHE ANORDNUNG ZUM ERFASSEN VON LICHT**

LIGHT DETECTING OPTICAL DEVICE

DISPOSITIF OPTIQUE POUR LA DETECTION DE LUMIERE

(84) Benannte Vertragsstaaten:
**CH DE FR GB IT LI SE**

(30) Priorität: **12.03.1998  DE 19810615**

(43) Veröffentlichungstag der Anmeldung:
**21.02.2001  Patentblatt 2001/08**

(73) Patentinhaber:
• **Ruckstuhl, Thomas**
**93053 Regensburg (DE)**
• **Seeger, Stefan**
**93077 Bad Abbach a.d. Donau (DE)**

(72) Erfinder:
• **Ruckstuhl, Thomas**
**93053 Regensburg (DE)**

• **Seeger, Stefan**
**93077 Bad Abbach a.d. Donau (DE)**

(74) Vertreter: **Köllner & Kewitz**
**Patentanwälte**
**Robert-Bosch-Strasse 7**
**64293 Darmstadt (DE)**

(56) Entgegenhaltungen:
**WO-A-97/35181       DE-A- 3 626 724**
**DE-A- 19 651 935**

• **ENDERLEIN J, RUCKSTUHL T, SEEGER S: "Highly efficient optical detection of surface-generated fluorescence" APPLIED OPTICS, Bd. 38, Nr. 4, 1. Februar 1999, Seiten 724-732, XP002107573**

**Beschreibung**

Die Erfindung bezieht sich auf eine optische Anordnung zum Erfassen von Licht, das von einer Probe emittiert wird, nach dem Oberbegriff des Patentanspruchs 1.

Der Nachweis von Molekülen mit Hilfe von optischen Biosensoren geschieht in der Regel über den Nachweis einer spezifischen Bindung zwischen den Molekülen und sogenannten Rezeptormolekülen, die an einer Oberfläche immobilisiert sind. Der Nachweis von Molekülen kann über eine solche spezifische Bindung auch aus einem Gemisch sehr selektiv durchgeführt werden. Typische Rezeptormoleküle sind Antikörper oder DNS-Moleküle. Typische Oberflächen finden sich an optischen Fasern oder transparenten Objektträgern bzw. Mikroskop-Deckplättchen.

Häufig wird zum Nachweis der Moleküle ein für die Bindung zwischen gesuchtem Molekül und Rezeptormolekül spezifisches Fluoreszenzsignal detektiert. Dieses kann von den gesuchten Molekülen selbst erzeugt werden, sofern sie fluoreszenzfähig sind.

In der Regel wird jedoch ein sogenannter Sandwich-Test durchgeführt, bei dem das gesuchte Molekül selbst nicht fluoreszenzfähig ist, jedoch ein drittes, fluoreszenzmarkiertes sogenanntes Sondenmolekül selektiv an den Bindungskomplex aus gesuchtem Molekül und Rezeptormolekül bindet, nachdem sich dieser Komplex gebildet hat.

Um quantitative Aussagen über die Menge der an die Rezeptormoleküle gebundenen gesuchten Moleküle machen zu können, darf nur dasjenige Fluoreszenzlicht detektiert werden, das auf einen Bindungskomplex zwischen gesuchtem Molekül und Rezeptormolekül hinweist. Ein solcher Komplex befindet sich stets nahe derjenigen Oberfläche, auf der die Rezeptormoleküle immobilisiert sind. Daraus ergibt sich die Meßaufgabe, selektiv Fluoreszenzsignale von oberflächennah gebundenen Molekülen, also aus oberflächennahen Schichten, nachzuweisen.

In letzter Zeit hat sich die Erfassung oberflächennaher Fluoreszenz mit Hilfe der Detektion der sogenannten evaneszenten Strahlung in optischen Sensoren bewährt. Dabei wird das folgende physikalische Prinzip ausgenutzt.

Bei Auftreffen eines Lichtstrahls auf eine Grenzfläche von einem optisch dichten zu einem optisch dünneren Medium kann es zu Totalreflexion kommen, wenn der Einfallswinkel des Lichtstrahls bezogen auf die Grenzflächennormale größer als der Grenzwinkel der Totalreflexion $\alpha$ ist, der sich aus

$$\sin \alpha = \frac{n_2}{n_1} \qquad (1)$$

bestimmt ($n_2$ = Brechungsindex des optisch dünneren Mediums und $n_1$ = Brechungsindex des optisch

dichteren Mediums). Lichtstrahlen, die unter einem Winkel auf die Grenzfläche treffen, der größer als $\alpha$ ist, können gemäß strahlenoptischer Betrachtung das optisch dichtere Medium nicht verlassen.

Umgekehrt ist es gemäß der Strahlenoptik nicht möglich, einen Lichtstrahl vom optisch dünneren in das optisch dichtere Medium einzustrahlen, wenn er sich im optisch dichteren Medium unter einem Winkel zur Grenzfläche ausbreitet, der größer als der Grenzwinkel der Totalreflexion $\alpha$ ist.

Eine genauere wellenoptische Betrachtung zeigt jedoch, daß die Feldstärke der aus dem dichten Medium auf die Grenzfläche auftreffenden und totalreflektierten Lichtwelle an der Grenzfläche zum optisch dünneren Medium nicht sprunghaft auf Null abfällt, sondern von ihrem Wert im optisch dichten Medium exponentiell mit dem Abstand von der Grenzfläche abfällt. Typische Abklingkonstanten für diesen exponentiellen Abfall der Feldstärke liegen in der Größenordnung der Wellenlänge des eingestrahlten Lichts. Trotz Totalreflexion tritt also ein gewisser Anteil des Lichts in das optisch dünnere Medium ein.

Ebenso gilt, daß Licht von einem Ort im optisch dünneren Medium, der nahe an der Grenzfläche liegt, auch in das optisch dichtere Medium unter einem Winkel eintreten kann, der größer als der Grenzwinkel der Totalreflexion $\alpha$ ist.

Bei optischen Fasersensoren wird dieser Effekt ausgenutzt. Bei diesen Sensoren sind auf der Faseroberfläche Rezeptormoleküle immobilisiert. Anregungslicht, in der Regel Laserlicht, wird in die Faser eingekoppelt. Das Laserlicht wird in der Faser durch Totalreflexion weitergeleitet. Es passiert auch die Bereiche der Faser, an deren Oberfläche die fluoreszenzfähigen Moleküle gebunden sind. Aufgrund des evaneszenten Feldes des Anregungslichts an der Oberfläche der Faser können fluoreszenzfähige Moleküle angeregt werden. Der Abstand der Moleküle von der Faseroberfläche beträgt im gebundenen Zustand lediglich wenige Nanometer, so daß sich der exponentielle Abfall der Feldstärke des Anregungslichts kaum bemerkbar macht. Die an die Oberfläche gebundenen Moleküle werden daher effektiv durch das Anregungslicht angeregt. Das von den Molekülen emittierte Licht kann ebenfalls wieder in die Faser einkoppeln, teilweise unter Winkeln, bei denen das einmal eingekoppelte Fluoreszenzlicht aufgrund von Totalreflexion durch die Faser weitergeleitet wird. An einem der Faserenden kann dann der weitergeleitete Fluoreszenzlichtanteil der gebundenen Moleküle nachgewiesen werden.

Nachteilig an optischen Fasersensoren, die evaneszente Strahlung erfassen, ist, daß die Effizienz, mit der sie emittierte Fluoreszenzphotonen sammeln (einfangen und weiterleiten) können, sehr begrenzt ist. Insbesondere reicht die Empfindlichkeit einer solchen Anordnung nicht zum Nachweis einzelner fluoreszenzfähiger Moleküle aus.

Aufgabe der Erfindung ist es, die Lichtsammeleffizienz für evaneszent in ein optisch dichtes Medium eingekoppeltes Licht zu verbessern.

Diese Aufgabe wird erfindungsgemäß durch eine optische Anordnung mit den Merkmalen des Patentanspruchs 1 gelöst.

Die erfindungsgemäße optische Anordnung kann sowohl Licht erfassen, das vor einer Grenzfläche erzeugt wurde, beispielsweise aufgrund von Chemolumineszenz, als auch vor der Grenzfläche gestreutes Licht, beispielsweise Fluoreszenzlicht infolge der Anregung durch eine Lichtquelle.

Das solcherart erzeugte Licht wird von einer Lichtsammeleinrichtung gesammelt. Diese kann außer dem erfindungsgemäßen Lichtleitkörper auch weitere Linsen, Spiegel, Filter und sonstige übliche optische Komponenten aufweisen.

Das von der Lichtsammeleinrichtung gesammelte Licht wird von der Detektionseinrichtung erfaßt. Auch diese kann ihrerseits wiederum außer einem üblichen Detektor weitere Linsen oder Filter zum Lenken des Lichts auf den Detektor aufweisen.

Die erste Stirnfläche kann vollständig eben sein, oder auch nur in einem Bereich eben sein, in dem die Probe angeordnet ist. Die Grenzfläche, vor der die Probe angeordnet ist, kann unmittelbar von der ersten Stirnfläche selbst gebildet werden. Alternativ kann die erste Stirnfläche optisch an eine Grenzfläche zwischen Probenmedium und optisch dichtem Medium gekoppelt sein. Das optisch dichte Medium kann in diesem Fall das Medium eines Objektträgers sein, der mit Hilfe eines optisch dichten Immersionsöls an die erste Stirnfläche gekoppelt ist. In letzterem Fall sollte der Durchmesser des Probenträgers und sollten die Abmessungen der ersten Stirnfläche derart gewählt sein, daß nicht durch rein geometrische Beschränkungen von der Probe emittiertes und in den dem optisch dichteren Medium zugewandten Halbraum abgestrahltes Licht am Eintritt in die erste Stirnfläche gehindert wird.

Wird Licht nahe der Grenzfläche von der Probe emittiert, beispielsweise durch Erzeugen von Fluoreszenzlicht, so tritt ein Teil des Lichts durch die erste Stirnfläche in den Lichtleitkörper ein und breitet sich unter einem Winkel (bezogen auf die Flächennormale der ersten Stirnfläche am Ort der Probe im Lichtleitkörper) aus, der größer als der dem Brechungsindexverhältnis des optisch dichten Mediums und des Probenmediums entsprechende Grenzwinkel der Totalreflexion ist. Diese sogenannte evaneszente Strahlung konnte bei Fasersensoren nur teilweise zum Faserausgang und damit auf einen Detektor geleitet werden. Erfindungsgemäß sorgt die Anordnung und Ausbildung der Mantelfläche dafür, daß die evaneszente Strahlung an der Mantelfäche vollständig in den Lichtleitkörper zurückgespiegelt wird. Dies kann einerseits im Wege der Totalreflexion geschehen, andererseits aber auch durch eine entsprechende Verspiegelung der Mantelfläche. Die evaneszente Strahlung kann dann in bekannter Weise auf eine Detektionseinrichtung gelenkt und von dieser erfaßt werden.

Auf diese Weise kann der evaneszente Strahlungsanteil im wesentlichen vollständig erfaßt werden. Da auch klassische Strahlung, d.h. Licht, das sich unter einem Winkel (bezogen auf die oben erwähnte Plächennormale) im Lichtleitkörper ausbreitet, der kleiner als der dem Brechungsindexverhältnis des optisch dichteren und des Probenmediums entsprechende Grenzwinkel der Totalreflexion ist, erfaßt werden kann, kann somit nahezu die gesamte Strahlung eines Halbraums erfaßt werden.

Bei einer vorteilhaften Weiterbildung ist die erste Stirnfläche eben. Die an diese angrenzende Mantelfläche ist derart gegenüber der ersten Stirnfläche geneigt, daß das von der Probe emittierte Licht an der Mantelfläche totalreflektiert wird. Dadurch kann eine verlustfreie Reflexion erreicht werden. Ferner erübrigt sich eine spezielle Verspiegelung der Mantelfläche.

Bei einer vorteilhaften Weiterbildung der Erfindung sind die Anordnung und Ausbildung der Mantelfläche derart gewählt, daß im wesentlichen ausschließlich der evaneszente Anteil auf die Mantelfläche trifft bzw. an ihr totalreflektiert wird. Die klassische Strahlung verläßt hingegen den Lichtleitkörper unreflektiert.

Dies erlaubt eine äußerst effiziente Trennung von evaneszenter und klassischer Strahlung. Evaneszente Strahlungsanteile können nur von grenzflächennahen Strahlungsquellen herrühren. Dadurch kann sehr selektiv das oberflächennah emittierte Licht einer Probe detektiert werden, wie es für optische Biosensoren essentiell ist.

Bei einer vorteilhaften Weiterbildung der Erfindung ist der Lichtleitkörper rotationssymmetrisch um eine Achse ausgebildet, und die Probe ist auf oder in unmittelbarer Nähe der Achse angeordnet. Die Mantelfläche kann dann ein Ausschnitt eines Rotationsparaboloids sein, wobei die Probe nahe dem Brennpunkt angeordnet ist. Stammt das emittierte Licht aus der Probe und damit dem Brennpunkt des Rotationsparaboloids, so wird es durch Reflexion an der Mantelfläche parallelisiert. Das so parallelisierte Licht kann in einfacher Weise gesammelt und detektiert werden. Ist die zweite Stirnfläche eben, so tritt das parallelisierte Licht auch parallel aus dem Lichtleitkörper aus. Es kann dann besonders einfach weitergeleitet werden. Darüber hinaus hängt der radiale Abstand des aus der zweiten Stirnfläche austretenden Lichtstrahls in wohldefinierter Weise vom Eintrittswinkel in die erste Stirnfläche ab.

In einer vorteilhaften Weiterbildung der Erfindung ist die Mantelfläche ein Ausschnitt eines Rotationsellipsoids, wobei die Probe nahe einem Brennpunkt des Rotationsellipsoids auf oder vor der ersten Stirnfläche angeordnet ist.

Diese Weiterbildung ist besonders gut geeignet, klassische und evaneszente Strahlung im zweiten Brennpunkt des Rotationsellipsoids jenseits der zweiten Stirnfläche des Lichtleitkörpers zusammenführen und zu erfassen. Dadurch kann auf elegante Weise eine maximale Lichtsammeleffizienz an einem nahezu punktförmigen Sensor erreicht werden.

Die Mantelfläche kann auch als Kegelstumpfaußenfläche ausgebildet sein. Dies gestattet eine einfache Herstellung des Lichtleitkörpers. Außerdem hat sich gezeigt, daß eine kegelstumpfförmige Mantelfläche im Vergleich zu einer rotationsparaboloidförmigen Mantelfläche zu einer verbesserten Abbildungsqualität führt, da Abweichungen des Orts der Strahlungsquelle von der Achse geringere Auswirkungen auf die Abbildungsschärfe haben.

Bei einem anderen Ausführungsbeispiel ist die zweite Stirnfläche konvex ausgebildet und dient für das totalreflektierte Licht als Sammellinse. Es kann so direkt auf einen Punkt fokussiert werden. In diesem Fokuspunkt kann sich beispielsweise die Detektionseinrichtung befinden. Dies gestattet den Aufbau der Lichtsammeleinrichtung, die im wesentlichen aus dem Lichtleitkörper besteht.

Bei einer vorteilhaften Weiterbildung der Erfindung ist zwischen der zweiten Stirnfläche und der Detektionseinrichtung eine lichtabsorbierende Blende derart angeordnet, daß sie selektiv den klassischen Strahlungsanteil des aus dem Lichtleitkörper austretenden Lichts absorbiert. Bei den meisten Ausbildungen der Mantelfläche treten evaneszente und klassische Strahlungsanteile an unterschiedlichen Orten und/oder unter unterschiedlichen Winkeln aus der zweiten Stirnfläche aus. Eine Blende ist dann in der Lage, aus einem Gemisch von klassischer und evaneszenter Strahlung selektiv die klassische Strahlung zu absorbieren und nur die evaneszente Strahlung passieren zu lassen. Ein selektiver Nachweis ausschließlich der evaneszenten Strahlung erlaubt wiederum den für optische Biosensoren wichtigen selektiven Nachweis von grenzflächennah emittiertem Licht.

Alternativ dazu kann die Detektionseinrichtung derart ausgebildet sein, das sie klassische und evaneszente Strahlung getrennt detektiert. Dies kann beispielsweise mit Hilfe eines örtlich auflösenden Detektors geschehen. Der getrennte Nachweis von evaneszenter und klassischer Strahlung erlaubt eine weitere Differenzierung zwischen oberflächennah und oberflächenfern gestreutem Licht. Auch kann damit der Abstand der Probe von ersten Grenzfläche ermittelt werden.

Für den Nachweis von Fluoreszenzlicht aus einer Probe weist die optische Anordnung eine Lichtquelle zum Bestrahlen der Probe auf. Das Licht kann dabei durch den Lichtleitkörper hindurch auf die Probe geleitet werden, indem es beispielsweise an der zweiten Stirnfläche eingestrahlt wird oder derart eingestrahlt wird, daß es die Probe evaneszent anregt. In der Regel wird es jedoch probenseitig eingestrahlt und gelangt damit durch die erste Stirnfläche in den Lichtleitkörper. Zwischen Lichtleitkörper und Detektionseinrichtung ist bei einer vorteilhaften Weiterbildung eine Absorptionseinrichtung angeordnet, die das Licht der Lichtquelle absorbiert, so daß es den Detektor nicht erreicht. Auf diese Weise wird ein störender Einfluß des Lichts der Lichtquelle auf die Detektion vermieden. Dies ist essentiell für das Erreichen der gewünschten extrem hohen Empfindlichkeiten, die auch einen Nachweis einzelner Fluoreszenzmoleküle erlauben.

Bei einer Weiterbildung der Erfindung ist zwischen der ersten Stirnfläche und der Grenzfläche eine dünne Trennscheibe aus einem optisch dichteren Medium angeordnet. Dies kann typischerweise ein Objektträger oder ein Mikroskop-Deckplättchen sein. Die Dikke der Trennscheibe ist wesentlich geringer als die Minimalabmessung der ersten Stirnfläche, so daß der überwiegende Anteil der evaneszenten Strahlung in den Lichtleitkörper eintreten kann. Dies ist besonders günstig für eine routinemäßige Durchführung von Analysen. Typischerweise werden dabei Rezeptormoleküle auf einer Probenträger-Oberfläche immobilisiert. An der gegenüberliegenden Oberfläche wird der Probenträger durch ein Immersionsöl optisch mit der ersten Stirnfläche gekoppelt. Auf diese Weise werden ein direkter Kontakt zwischen dem Lichtleitkörper und der Probe und damit störende Verunreinigungen der ersten Stirnfläche und dadurch hervorgerufene Untergrundsignale vermieden.

Bei einer vorteilhaften Weiterbildung der Erfindung ist der Probenträger gegenüber dem Lichtleitkörper parallel zur ersten Stirnfläche verschiebbar gelagert. Die optische Kopplung zwischen dem Lichtleitkörper und dem Probenträger bleibt durch das Immersionsöl auch bei einer Verschiebung des Probenträgers erhalten. Damit ist es möglich, die Oberfläche eines Probenträgers daraufhin abzusuchen, ob ein einzelnes oder wenige einzelne gesuchte Moleküle an die auf ihm immobilisierten Rezeptormoleküle gebunden haben. Dies ist wesentlich, wenn extreme Empfindlichkeiten erreicht werden sollen, da die Konzentrationen der gesuchten Moleküle derart gering sind, daß in einem vorgegebenen kleinen Beobachtungsbereich kein gesuchtes Molekül an ein Rezeptormolekül gebunden ist.

Bei einer vorteilhaften Weiterbildung der Erfindung ist der Probenträger Teil einer Durchflußzelle. Dies erlaubt eine Automatisierung von Messungen und das Analysieren von kontinuierlichen Probenströmen.

Vorteilhafte Weiterbildungen der Erfindung sind in den Unteransprüchen gekennzeichnet.

Im folgenden wird die Erfindung anhand von in der Zeichnung dargestellten Ausführungsbeispielen näher erläutert. In der Zeichnung zeigt:

Fig. 1    eine Schnittansicht eines erfindungsgemäßen Lichtleitkörpers mit einer rotationsparaboloidförmigen Mantelfläche;

Fig. 2    den Lichtleitkörper gemäß Fig. 1 mit einer konvexen und als Sammellinse wirkenden zweiten Stirnfläche;

Fig. 3    eine Schnittansicht eines erfindungsgemäßen Lichtleitkörpers mit einer rotationsellipsoidförmigen Mantelfläche;

Fig. 4 eine Schnittansicht einer erfindungsgemäßen optischen Anordnung; und

Fig. 5 schematisch ein Ausführungsbeispiel der erfindungsgemäßen optischen Anordnung, das aus einem Array von einzelnen optischen Anordnungen gebildet ist.

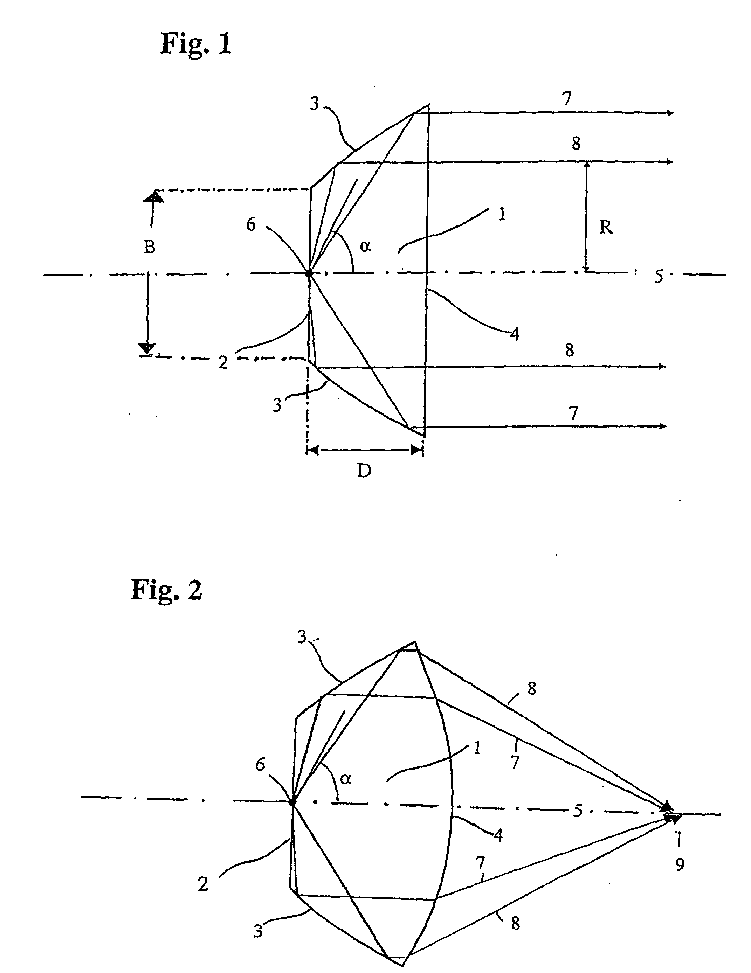

Fig. 1 zeigt einen Lichtleitkörper 1 mit einer ebenen ersten Stirnfläche 2, einer rotationsparaboloidförmigen Mantelfläche 3 und einer ebenen zweiten Stirnfläche 4. Der Lichtleitkörper ist rotationssymmetrisch bezogen auf eine Achse 5 ausgebildet.

Ein typisches Material für den Lichtleitkörper ist Glas mit einem Brechungsindex von 1,5. Die Probe befindet sich üblicherweise in einer wäßrigen Lösung mit einem Brechungsindex von ca. 1,3. Andere Materialien bzw. Medien sind jedoch ebenfalls denkbar. Der Lichtleitkörper könnte beispielsweise von einem flüssigen Medium oder einem anderen Festkörper (Kunststoff/Glas-Grenzfläche) umgeben sein.

Licht, das aus einer Probe 6 in den Lichtleitkörper 1 eintritt, umfaßt zunächst die sogenannte "klassische" Strahlung 7. Die klassische Strahlung tritt unter einem Winkel (bezogen auf die Achse 5) in den Lichtleitkörper 1 ein, der kleiner als der dem Brechungsindexverhältnis des Lichtleitkörpermaterials und des Probenmediums entsprechende Grenzwinkel der Totalreflexion $\alpha$ ist.

Ferner können Lichtstrahlen unter einem Winkel eintreten, der größer als der Grenzwinkel der Totalreflexion $\alpha$ im Lichtleitkörper 1 ist. Diese Strahlen werden evaneszente Strahlen 8 genannt.

Der Abstand R zwischen den an der Mantelfläche reflektierten Lichtstrahlen 7 bzw. 8 und der Achse 5 hängt nur vom Winkel ab, unter dem der jeweilige Lichtstrahl in den Lichtleitkörper 1 ausgehend von der Probe 6 eintritt. Daher läßt sich aus der durch die zweite Stirnfläche 4 austretenden Strahlung mit Hilfe eines ortsauflösenden Detektors, beispielsweise einer CCD-Kamera, die Winkelabstrahl-Charakteristik der Probe rekonstruieren.

Mit einem solchen ortsauflösenden Detektor lassen sich ferner die evaneszente und die klassische Strahlung gleichzeitig detektieren und selektiv analysieren. Dadurch können zusätzliche Informationen gewonnen werden, z.B. über den Abstand der Probe von einer Grenzfläche.

Der Durchmesser B der ersten Stirnfläche ist frei wählbar. Der genaue Verlauf der zum Rotationsparaboloid gehörenden Parabel kann für den Fall, daß Probe und Brennpunkt im Effekt auf der ersten Stirnfläche angeordnet sind, beschrieben werden durch

$$y = -\frac{B}{4} + \frac{1}{B} x^2 , \qquad (2)$$

wobei

x der Abstand eines Punktes (x, y) auf der Parabel von der Achse 5 und

y der Abstand des Punktes von der Ebene der ersten Stirnfläche ist.

Typische Werte für B liegen im Bereich von 1 bis 5 cm. Jedoch sind auch miniaturisierte Formen des Lichtleitkörpers 1 denkbar mit sehr kleinen Werten für B, beispielsweise kleiner als 100 µm.

Die Dicke D des Lichtleitkörpers ist bei dem in Fig. 1 gezeigten Ausführungsbeispiel so gewählt, daß sowohl evaneszente Strahlung 8 als auch klassische Strahlung 7 an der rotationsparaboloidförmigen Mantelfläche 3 total reflektiert wird.

Um ausschließlich die gesamte evaneszente Strahlung aus der Probe zu sammeln, können die Abmessungen des Lichtleitkörpers 1 derart gewählt werden, daß keine klassische Strahlung auf die Mantelfläche trifft und total reflektiert wird. Dies kann dadurch erreicht werden, daß die Dicke D des Lichtleitkörpers 1 entsprechend gering gewählt wird. Der minimale Winkel, unter dem evaneszente Strahlung bezogen auf die Rotationssymmetrieachse sich im Lichtleitkörper 1 ausbreitet, ergibt sich aus Gleichung (1) unter Berücksichtigung, daß $n_2$ der Brechungsindex des Probenmediums (typischerweise Wasser mit einem Brechungsindex von 1,3) und $n_1$ der Brechungsindex des Lichtleitkörpers (typischerweise der von Glas: 1,5) ist. Daraus ergibt sich ein $\alpha$ von ca. 69°. Der Schnittpunkt einer Gerade, die ausgehend von der Probe unter 69° bezogen auf die Achse 5 verläuft, mit der oben angegebenen Parabel y legt die Dicke D eines ausschließlich evaneszente Strahlung reflektierenden Lichtleitkörpers fest.

Fig. 2 zeigt den Lichtleitkörper gemäß Fig. 1, jedoch nicht mit einer ebenen zweiten Stirnfläche 4, sondern mit einer sphärisch konvexen zweiten Stirnfläche 4. Von der Probe 6 in den Lichtleitkörper 1 eintretendes Licht wird bei einer Reflexion an der rotationsparaboloidförmigen Mantelfläche 3 parallelisiert, sofern sich die Probe 6 im Brennpunkt des Rotationsparaboloids befindet. Die dabei entstehenden parallelen Lichtstrahlen 7 und 8 werden durch die sphärisch konvexe zweite Stirnfläche 4 auf einen Punkt 9 fokussiert.

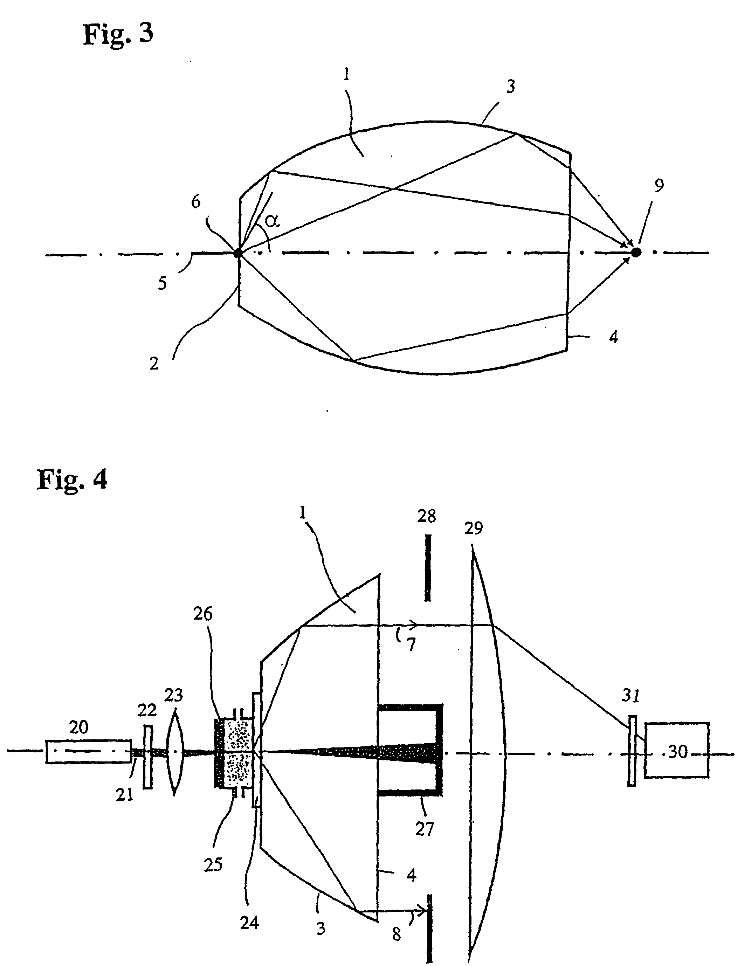

Fig. 3 zeigt einen Lichtleitkörper 1 mit einer ebenen ersten Stirnfläche 2, einer ebenen zweiten Stirnfläche 4, und einer rotationsellipsoidförmigen Mantelfläche 3. Die Probe 6 befindet sich in einem der beiden Brennpunkte des zugehörigen Rotationsellipsoids. Von der Probe 6 emittiertes Licht, das in den Lichtleitkörper 1 eintritt, wird zum größten Teil an der rotationsellipsoidförmigen Mantelfläche 3 totalreflektiert und im zweiten Ellipsen-Brennpunkt fokussiert. Da der zweite Brennpunkt im hier gezeigten Ausführungsbeispiel außerhalb des Lichtleitkörpers 1 liegt, wird das Licht beim Austritt aus der ebenen zweiten Stirnfläche 4 in Richtung auf die Achse 5 hin gebrochen. Der Fokus 9 liegt daher etwas näher am Lichtleitkörper 1 als der zweite Brennpunkt des Rotationsellipsoids.

Die zweite Stirnfläche könnte ebensogut sphärisch konvex ausgebildet sein. Dies hätte zur Folge, daß der Fokus 9 noch näher am Lichtleitkörper 1 liegt.

Bildet man die zweite Stirnfläche 4 sphärisch konkav aus, wobei der Mittelpunkt der zugehörigen Kugel mit dem zweiten Ellipsen-Brennpunkt zusammenfällt, so treten alle Strahlen, die an der rotationsellipsoidförmigen Mantelfläche 3 reflektiert wurden, ungebrochen durch die zweite Stirnfläche 4 aus, und der Fokus 9 und der zweite Brennpunkt des Rotationsellipsoids fallen zusammen.

Fig. 4 zeigt eine optische Anordnung zum Erfassen von von einer Probe emittiertem Licht. Aus einer Lichtquelle 20, die beispielsweise ein Diodenlaser sein kann, tritt ein Lichtstrahl 21 aus. Dieser wird zunächst durch einen optischen Filter 22 spektral bereinigt, da die Strahlung von Diodenlasern in der Regel spektrale Komponenten enthält, die nicht mit der gewünschten Wellenlänge der Laserstrahlung übereinstimmen. Anschließend wird der Laserstrahl durch eine Sammellinse 23 auf die Probe 6 fokussiert. Als Sammellinse kann auch ein Mikroskopobjektiv dienen.

Die Probe 6 befindet sich an der Oberfläche eines Objektträgers 24. Die gegenüberliegende Oberfläche des Objektträgers ist durch ein Immersionsöl optisch mit dem Lichtleitkörper 1 gekoppelt. Der Objektträger 24 bildet eine Wand einer Durchflußzelle 25. Die dem Objektträger 24 gegenüberliegende Wand 26 der Durchflußzelle 25 weist ein für die Anregungswellenlänge transparentes Fenster auf.

Auf dem Objektträger 24 können beispielsweise Antikörper immobilisiert sein. In die Durchflußzelle wird nun eine die gesuchten Moleküle bzw. Antigene enthaltende Lösung eingelassen. Das Medium in der Probenzelle kann anstelle einer Flüssigkeit auch ein Gas sein, mit dem die gesuchten Moleküle gemischt sind.

Ziel der Analyse ist es, die Konzentration der Antigene oder deren Vorhandensein festzustellen. Zu diesem Zweck enthält die Lösung außerdem zweite Antiköper, sogenannte Sondenmoleküle, die selektiv an den Komplex aus erstem Antikörper und daran gebundenem Antigen binden. Der erste Antikörper ist dabei selektiv für das Antigen, d.h. er geht eine sehr spezifische Bindung fast nur mit dem Antigen ein. Das Sondenmolekül ist fluoreszenzmarkiert. Der Fluoreszenzfarbstoff muß dabei derart gewählt werden, daß seine Absorptionswellenlänge mit der Emissionswellenlänge der Lichtquelle 20 übereinstimmt. Handelt es sich bei der Lichtquelle um einen Diodenlaser mit einer Emissonswellenlänge von beispielsweise ca. 630 nm, so bietet sich als Farbstoff ein Rhodamin- oder Cyanin-Farbstoff an, z.B. Cy5. Typische zu detektierende Antigene sind Tumormarker.

Nach Durchtritt durch die Probe 6, den Objektträger 24, das Immersionsöl und den Lichtleitkörper 1 tritt der Laserstrahl 21 durch die zweite Stirnfläche 4 wieder aus dem Lichtleitkörper 1 aus. Er wird dort von einem Absorber 27 aufgefangen.

Die von der Probe 6 emittierte klassische Strahlung 8 wird von einer ringförmigen Blende 28 absorbiert. Die von der Probe emittierte evaneszente Strahlung 7 gelangt auf eine Sammellinse 29. Da die Mantelfläche 3 bei diesem Beispiel rotationsparaboloidförmig gewählt ist und so ausgebildet ist, daß die an der Oberfläche des Objektträgers 24 immobilisierte Probe sich im Brennpunkt des Rotationsparaboloids befindet, verläßt die evaneszente Strahlung 7 den Lichtleitkörper 1 weitestgehend parallelisiert.

Die Sammellinse 29 lenkt daher die evaneszente Strahlung 7 auf den Fokus der Sammellinse 29, bei dem sich die Detektionseinrichtung 30 befindet.

Diese kann beispielsweise eine zum Einzelphotonenzählen geeignete Avalanche-Photodiode, eine Silizium-PIN-Diode, ein Photomultiplier oder ein CCD-Chip sein. Vor der Detektionseinrichtung 30 befindet sich ein weiterer optischer Filter 31, in der Regel ein Interferenzfilter, der selektiv nur das Fluoreszenzlicht der mit den Sondenmolekülen gekoppelten Farbstoffe passieren läßt. Der Interferenzfilter sperrt insbesondere Licht von der Wellenlänge der Lichtquelle 20, das beispielsweise im Bereich der Probe 6 gestreut wurde.

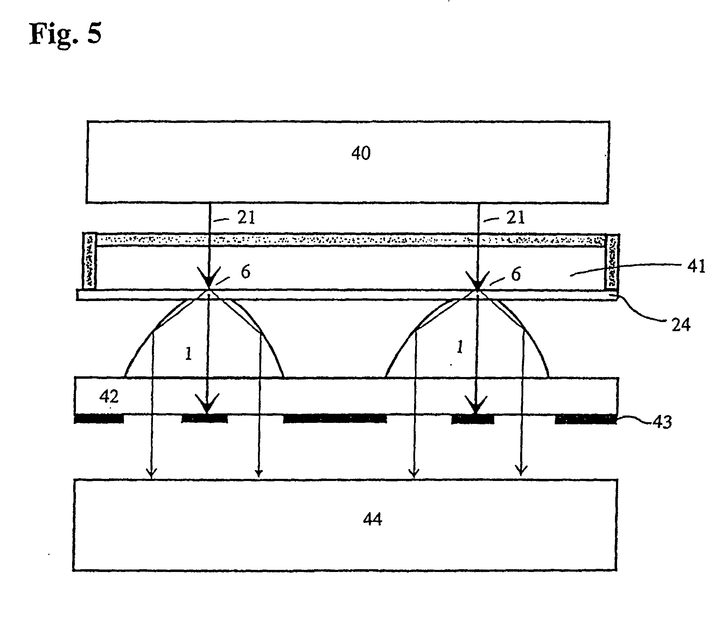

Fig. 5 zeigt ein Ausführungsbeispiel der optischen Anordnung, bei dem mehrere Anregungsstrahlen 21, mehrere Proben 6 und mehrere Lichtsammeleinrichtungen mit jeweils einem Lichtleitkörper 1 verbunden mit mehreren Detektionseinrichtungen prinzipiell dargestellt sind. Bei der schematisch dargestellten Einrichtung 40 wird eine Mehrzahl von Lichtstrahlen 21 erzeugt. Dies kann entweder durch eine Mehrzahl von Lichtquellen oder durch Aufteilen einer oder weniger Lichtquellen in mehrere Lichtstrahlen und anschließendes Fokussieren dieser Lichtstrahlen beispielsweise mit einem Linsen- oder Mikrolinsenarray geschehen.

Die Lichtstrahlen 21 treten in eine Probenzelle 41 ein. Die Probenzelle 41 wird an einer Seite durch einen Objektträger 24 begrenzt. Auf dem Objektträger sind in üblicher Weise Rezeptormoleküle immobilisiert. Der Objektträger ist mit Hilfe von Immersionsöl an eine Mehrzahl von Lichtleitkörpern 1 optisch gekoppelt. Diese werden von einem transparenten Träger 42 getragen. Auf der Unterseite des transparenten Trägers 42 ist eine Absorbermaske 43 zum Absorbieren einerseits der Lichtstrahlen 21 und andererseits der klassischen Strahlung angeordnet.

Unterhalb des transparenten Trägers 42 befindet sich eine Detektionseinrichtung 44, die beispielsweise der Chip einer CCD-Kamera sein kann. Mit Hilfe einer solchen CCD-Kamera kann der zeitliche Bindungsverlauf in den einzelnen Proben 6 gleichzeitig verfolgt werden.

Ein solches Ausführungsbeispiel erlaubt eine Parallelisierung von Meßpunkten für einen Biosensor. Damit kann einerseits eine Beschleunigung von Analysen erreicht werden. Andererseits können in den verschiedenen Proben an der Oberfläche des Probenträgers 24 unterschiedliche Rezeptormoleküle immobilisiert sein, so daß mehrere Analysen hinsichtlich unterschiedlicher gesuchter Moleküle gleichzeitig durchgeführt werden können. Ein solcher Test wird üblicherweise Multiparameter-Test genannt.

Bei dem Ausführungsbeispiel gemäß Fig. 5 kann ein erstes Linsenarray zum Erzeugen einer Vielzahl von Lichtstrahlen 21 mit einem zweiten Linsenarray als Teil der Lichtsammeleinrichtung kombiniert werden, wobei die Dimensionen der beiden Arrays abgestimmt sind auf die Dimensionen der einzelnen Pixel des Chips einer als Detektionseinrichtung dienenden CCD-Kamera.

Im Rahmen der Erfindung sind zahlreiche Abwandlungen möglich. So ist die Form der Mantelfläche 3 nicht auf Ausschnitte eines Rotationsparaboloids oder Rotationsellipsoids beschränkt. Möglich ist auch ein allgemeines Paraboloid, etwa ein im Querschnitt ellipsenförmiges, das das Licht der Probe auf einen Strich abbildet. Wichtig ist nur, daß evaneszente Strahlung an der Mantelfläche 3 möglichst vollständig reflektiert wird.

Die erste und die zweite Stirnfläche können sowohl parallel als auch winkelversetzt zueinander angeordnet sein.

Der Lichtleitkörper 1 kann außer den drei genannten Flächen 2, 3 und 4 in den Übergangsbereichen zwischen den Flächen weitere kleinflächige Bereiche aufweisen.

Als Lichtquellen 20 können sämtliche in der Spektroskopie gängigen Lichtquellen verwendet werden, d.h. insbesondere Lampen und Laser unterschiedlichster Art.

**Patentansprüche**

**1.** Optische Anordnung zum Erfassen von Licht, das von einer Probe (6) emittiert wird, mit wenigstens einem Lichtleitkörper (1), der eine erste Stirnfläche (2), eine Mantelfläche (3) und eine zweite Stirnfläche (4) aufweist, wobei die Probe in einem Probenmedium vor der ersten Stirnfläche (2) angeordnet ist und der wenigstens eine Lichtleitkörper (1) aus einem für das emittierte Licht gegenüber dem Probenmedium optisch dichteren Material besteht;
    **dadurch gekennzeichnet,**
    **daß** die erste Stirnfläche (2) zumindest in einem der Probe (6) benachbarten Bereich eben ist;
    **daß** die erste Stirnfläche (2) eine Grenzfläche bildet oder optisch an eine Grenzfläche zwischen dem Probenmedium und einem vor der ersten Stirnfläche (2) angeordneten, gegenüber dem Probenmedium optisch dichteren Medium angekoppelt ist, so daß im wesentlichen das gesamte von der Probe (6) emittierte und durch die Grenzfläche hindurch in den der ersten Stirnfläche (2) zugewandten Halbraum abgestrahlte Licht, einschließlich evaneszent abgestrahlten Lichts, das heißt oberhalb des Totalreflexionswinkels der Grenzfläche in den Halbraum abgestrahlten Lichts, durch die erste Stirnfläche (2) in den Lichtleitkörper (1) eintritt; und
    **daß** die Mantelfläche (3) und die zweite Stirnfläche (4) so angeordnet und ausgebildet sind, daß im wesentlichen der gesamte evaneszent abgestrahlte, in den Lichtleitkörper (1) eingekoppelte Lichtanteil des von der Probe (6) emittierten Lichts an der Mantelfläche (3) vollständig in den Lichtleitkörper (1) zurückgespiegelt, durch die zweite Stirnfläche (4) hindurchgeleitet und auf eine Detektionseinrichtung (30) gelenkt wird.

**2.** Optische Anordnung nach Anspruch 1, **dadurch gekennzeichnet, daß** zwischen der ersten Stirnfläche (2) und der Grenzfläche eine dünne Trennscheibe aus dem optisch dichteren Medium angeordnet ist, und wobei die Dicke der Trennscheibe wesentlich geringer als die Minimalabmessung der ersten Stirnfläche (2) ist.

**3.** Optische Anordnung nach Anspruch 1 oder 2, **dadurch gekennzeichnet, daß** die erste Stirnfläche (2) eben ist und die an die erste Stirnfläche (2) angrenzende Mantelfläche (3) derart gegenüber der ersten Stirnfläche geneigt ist, daß das von der Probe (6) emittierte Licht unter einem Winkel zur jeweiligen Oberflächennormale auf die Mantelfläche (3) trifft, der größer als der dem Brechungsindexverhältnis von Lichtleitkörpermaterial und Umgebungsmedium entsprechende Grenzwinkel der Totalreflexion ist.

**4.** Optische Anordnung nach einem der Ansprüche 1 bis 3, **dadurch gekennzeichnet, daß** die Mantelfläche (3) derart ausgebildet und angeordnet ist, daß im wesentlichen ausschließlich der evaneszent abgestrahlte, in den Lichtleitkörper (1) eingekoppelte Lichtanteil des von der Probe (6) emittierten Lichts auf die Mantelfläche (3) auftrifft.

**5.** Optische Anordnung nach einem der Ansprüche 1 bis 4, **dadurch gekennzeichnet, daß** der Lichtleitkörper (1) rotationssymmetrisch um eine Achse (5) ausgebildet ist, wobei die Probe (6) auf oder in unmittelbarer Nähe der Achse angeordnet ist.

**6.** Optische Anordnung nach Anspruch 5, **dadurch gekennzeichnet, daß** die Mantelfläche (3) ein Ausschnitt eines Rotationsparaboloids ist, wobei die Probe (6) nahe dem Brennpunkt vor der ersten Stirnfläche (2) angeordnet ist.

**7.** Optische Anordnung nach Anspruch 6, **dadurch gekennzeichnet, daß** die zweite Stirnfläche (4) eben ist, wobei das an der Mantelfläche (3) reflektierte Licht in parallelen Strahlen aus der zweiten Stirnfläche (4) austritt.

**8.** Optische Anordnung nach Anspruch 5, **dadurch gekennzeichnet, daß** die Mantelfläche (3) ein Ausschnitt eines Rotationsellipsoids ist, wobei die Probe (6) nahe einem Brennpunkt des Rotationsellipsoids vor der ersten Stirnfläche (2) angeordnet ist.

**9.** Optische Anordnung nach Anspruch 5, **dadurch gekennzeichnet, daß** die Mantelfläche (3) als Kegelstumpf ausgebildet ist.

**10.** Optische Anordnung nach Anspruch 6 oder 9, **dadurch gekennzeichnet, daß** die zweite Stirnfläche (4) konvex ausgebildet ist und für das totalreflektierte Licht als Sammellinse wirkt.

**11.** Optische Anordnung nach einem der Ansprüche 5 bis 10, **dadurch gekennzeichnet, daß** zwischen der zweiten Stirnfläche (4) und der Detektionseinrichtung (30) eine Licht absorbierende Blende (28) derart angeordnet ist, daß sie denjenigen Anteil des von der Probe (6) emittierten und durch den Lichtleitkörper (1) hindurchgeleiteten Lichts absorbiert, der in die erste Stirnfläche (2) unter einem Winkel gegenüber der Flächennormalen eintritt, der kleiner als der dem Brechungsindexverhältnis des Lichtleitkörpermaterials und des Probenmediums entsprechende Grenzwinkel der Totalreflexion ist (klassische Strahlung).

**12.** Optische Anordnung nach einem der Ansprüche 1 bis 11, **dadurch gekennzeichnet,**

**daß** sie eine Lichtquelle (20) zum Bestrahlen der Probe (6) aufweist; und

**daß** die Lichtquelle so angeordnet ist, daß ihr Licht probenseitig auf die erste Stirnfläche (2) eingestrahlt und nach Durchgang durch den Lichtleitkörper (1) von einer zwischen dem Lichtleitkörper (1) und der Detektionseinrichtung (30) angeordneten Absorptionseinrichtung (27) absorbiert wird.

**13.** Optische Anordnung nach Anspruch 2, **dadurch gekennzeichnet, daß** die Trennscheibe ein Probenträger (24) ist, der an der der ersten Stirnfläche (2) abgewandten Oberfläche mindestens eine Probe (6) trägt.

**14.** Optische Anordnung nach Anspruch 13, **dadurch gekennzeichnet, daß** der Probenträger (24) gegenüber dem Lichtleitkörper (1) parallel zur ersten Stirnfläche (2) verschiebbar gelagert ist.

**15.** Optische Anordnung nach Anspruch 13, **dadurch gekennzeichnet, daß** der Probenträger (24) Teil einer Durchflußzelle (25) ist.

**Claims**

**1.** Optical arrangement for detecting light emitted by a sample (6), having one or more optical waveguides (1) which possess a first end face (2), a shell surface (3), and a second end face (4), with the sample being arranged in a sample medium in front of the first end face (2), and the one or more optical-waveguide bodies (1) consisting of a material that has a higher refractive index than the sample medium for the light emitted;

**characterized in that**

the first end face (2) is flat, at least in a region near the sample (6);

the first end face (2) forms an interface, or is optically coupled to an interface between the sample medium and a medium located in front of the first end face (2) that has a higher refractive index than the sample medium, so that essentially the entire light emitted by the sample (6) and radiated through the interface into the half-space facing the first end face (2), including evanescently radiated light, i.e. light radiated into the half-space above the angle of total internal reflection, enters the optical-waveguide body (1) through the first end face (2); and

the shell surface (3) and the second end face (4) are arranged and constructed in such a way that essentially the entire evanescently radiated light radiated into the optical waveguide (1) is totally internally reflected at the shell surface (3) back into the optical waveguide (1), guided through the second end face (4), and directed onto a detection device (30).

**2.** Optical arrangement according to Claim 1, **characterized in that** a thin separating window of the medium with a higher refractive index is arranged between the first end face (2) and the interface, and the thickness of the separating window is considerably less than the minimum dimension of the first end face (2).

**3.** Optical arrangement according to Claim 1 or 2, **characterized in that** the first end face (2) is flat and the shell surface (3) adjoining the first end face (2) is at such an angle to the first end face that the light emitted by the sample (6) strikes the shell surface (3) at an angle to the respective surface normal which is larger than the critical angle of total internal reflection corresponding to the ratio of refractive indices of the optical-waveguide material and of the ambient medium.

**4.** Optical arrangement according to one of Claims 1 to 3, **characterized in that** the shell surface (3) is constructed and arranged in such a way that essentially only the evanescently radiated portion of the light emitted by the sample (6) and radiated into the optical waveguide (1) strikes the shell surface (3).

5. Optical arrangement according to one of Claims 1 to 4, **characterized in that** the optical waveguide (1) is constructed to be symmetrical about an axis (5), with the sample (6) being located on or in the immediate vicinity of the axis.

6. Optical arrangement according to Claim 5, **characterized in that** the shell surface (3) is a section of a paraboloid of revolution, with the sample (6) being located near to the focal point in front of the first end face (2).

7. Optical arrangement according to Claim 6, **characterized in that** the second end face (4) is flat, and the light reflected from the shell surface (3) emerges from the second end face (4) in parallel rays.

8. Optical arrangement according to Claim 5, **characterized in that** the shell surface (3) is a section of an ellipsoid of revolution, with the sample (6) being located near to a focal point of the ellipsoid of revolution in front of the first end face (2).

9. Optical arrangement according to Claim 5, **characterized in that** the shell surface (3) is constructed as a frustum of a cone.

10. Optical arrangement according to Claim 6 or 9, **characterized in that** the second end face (4) is of convex construction, and acts as a focusing lens for the totally internally reflected light.

11. Optical arrangement according to one of Claims 5 to 10, **characterized in that** a light-absorbing aperture (28) is arranged between the second end face (4) and the detection device (30) in such a way that it absorbs that portion of the light emitted by the sample (6) and propagated through the optical waveguide (1) which enters the first end face (2) at an angle to the surface normal that is smaller than the critical angle of total internal reflection corresponding to the ratio of refractive indices of the optical-waveguide material and of the sample medium (classical radiation).

12. Optical arrangement according to one of Claims 1 to 11, **characterized in that**
 it possesses a light source (20) for irradiating the sample (6); and that
 the light source is arranged in such a way that its light is radiated onto the first end face (2) from the sample side, and after passing through the optical waveguide (1) is absorbed by an absorption device (27) arranged between the optical waveguide (1) and the detection device (30).

13. Optical arrangement according to Claim 2, **characterized in that** the separating window is a microscopic slide (24) that holds one or more samples (6) on the surface facing away from the first end face (2).

14. Optical arrangement according to Claim 13, **characterized in that** the microscopic slide (24) is mounted in such a way that it can be shifted with respect to the optical waveguide (1) in parallel to the first end face (2).

15. Optical arrangement according to Claim 13, **characterized in that** the microscopic slide (24) is part of a flow cell (25).

**Revendications**

1. Dispositif optique pour la détection de lumière, qui est émise par un échantillon (6), avec au moins un corps conducteur de la lumière (1) qui présente une première face frontale (2), une surface latérale (3) et une seconde face frontale (4), l'échantillon étant disposé dans un milieu d'échantillon devant la première face frontale (2) et ledit au moins un corps conducteur de la lumière (1) se composant d'une matière optiquement plus dense pour la lumière émise que le milieu d'échantillon, **caractérisé en ce que** la première face frontale (2) est plane au moins dans une zone proche de l'échantillon (6); **en ce que** la première face frontale (2) forme une interface ou est couplée optiquement à une interface entre le milieu d'échantillon et un milieu optiquement plus dense que le milieu d'échantillon et disposé devant la première face frontale (2), de telle manière que sensiblement la totalité de la lumière émise par l'échantillon (6) est rayonnée à travers l'interface dans le demi-espace tourné vers la première face frontale (2), en ce compris la lumière rayonnée évanescente, c'est-à-dire la lumière rayonnée dans le demi-espace au-delà de l'angle de réflexion totale de l'interface, pénètre dans le corps conducteur de la lumière (1) à travers la première face frontale (2); et **en ce que** la surface latérale (3) et la seconde face frontale (4) sont disposées et configurées de telle manière que sensiblement la totalité de la fraction de lumière rayonnée évanescente, introduite dans le corps conducteur de la lumière (1), de la lumière émise par l'échantillon (6) soit entièrement réfléchie dans le corps conducteur de la lumière (1) par la surface latérale (3), transmise à travers la seconde face frontale (4) et dirigée vers un dispositif de détection (30).

2. Dispositif optique suivant la revendication 1, **caractérisé en ce qu'**un mince disque de séparation constitué du milieu optiquement plus dense est disposé entre la première face frontale (2) et l'interface, et dans lequel l'épaisseur du disque de séparation est sensiblement plus faible que la dimension minimale de la première face frontale (2).

3. Dispositif optique suivant la revendication 1 ou 2, **caractérisé en ce que** la première face frontale (2) est plane et la surface latérale (3) contiguë à la première face frontale (2) est inclinée par rapport à la première face frontale de telle manière que la lumière émise par l'échantillon (6) tombe sur la surface latérale (3) sous un angle, par rapport à la normale à la surface respective, qui est plus grand que l'angle limite de réflexion totale correspondant au rapport des indices de réfraction de la matière du corps conducteur de la lumière et du milieu ambiant.

4. Dispositif optique suivant l'une quelconque des revendications 1 à 3, **caractérisé en ce que** la surface latérale (3) est configurée et disposée de telle manière que sensiblement seule la fraction de lumière rayonnée évanescente, introduite dans le corps conducteur de la lumière (1), de la lumière émise par l'échantillon (6) tombe sur la surface latérale (3).

5. Dispositif optique suivant l'une quelconque des revendications 1 à 4, **caractérisé en ce que** le corps conducteur de la lumière (1) présente une configuration symétrique de révolution autour d'un axe (5), dans lequel l'échantillon (6) est disposé sur ou à proximité immédiate de l'axe.

6. Dispositif optique suivant la revendication 5, **caractérisé en ce que** la surface latérale (3) est une portion d'un paraboloïde de révolution, dans lequel l'échantillon (6) est disposé à proximité du foyer devant la première face frontale (2).

7. Dispositif optique suivant la revendication 6, **caractérisé en ce que** la seconde face frontale (4) est plane, dans lequel la lumière réfléchie sur la surface latérale (3) sort de la seconde face frontale (4) sous forme de rayons parallèles.

8. Dispositif optique suivant la revendication 5, **caractérisé en ce que** la surface latérale (3) est une portion d'un ellipsoïde de révolution, dans lequel l'échantillon (6) est disposé à proximité d'un foyer de l'ellipsoïde de révolution devant la première face frontale (2).

9. Dispositif optique suivant la revendication 5, **caractérisé en ce que** la surface latérale (3) est configurée en un tronc de cône.

10. Dispositif optique suivant la revendication 6 ou 9, **caractérisé en ce que** la seconde face frontale (4) est de forme convexe et agit comme une lentille convergente pour la lumière réfléchie totalement.

11. Dispositif optique suivant l'une quelconque des revendications 5 à 10, **caractérisé en ce qu'**un diaphragme (28) absorbant de la lumière est disposé entre la seconde face frontale (4) et le dispositif de détection (30), de telle manière qu'il absorbe la fraction de la lumière émise par l'échantillon (6) et transmise à travers le corps conducteur de la lumière (1) qui pénètre dans la première face frontale (2) sous un angle, par rapport à la normale à la surface, qui est plus petit que l'angle limite de réflexion totale (rayonnement classique) correspondant au rapport des indices de réfraction de la matière du corps conducteur de la lumière et du milieu d'échantillon.

12. Dispositif optique suivant l'une quelconque des revendications 1 à 11, **caractérisé en ce qu'**il présente une source de lumière (20) pour illuminer l'échantillon (6); et **en ce que** la source de lumière est disposée de telle manière que sa lumière soit envoyée sur la première face frontale (2) du côté de l'échantillon et qu'elle soit absorbée, après avoir traversé le corps conducteur de la lumière (1), par un dispositif d'absorption (27) disposé entre le corps conducteur de la lumière (1) et le dispositif de détection (30).

13. Dispositif optique suivant la revendication 2, **caractérisé en ce que** le disque de séparation est un porte-échantillons (24), qui porte au moins un échantillon (6) sur la surface située à l'opposé de la première face frontale (2).

14. Dispositif optique suivant la revendication 13, **caractérisé en ce que** le porte-échantillons (24) est logé de façon à pouvoir coulisser par rapport au corps conducteur de la lumière (1) parallèlement à la première face frontale (2).

15. Dispositif optique suivant la revendication 13, **caractérisé en ce que** le porte-échantillons (24) fait partie d'une cellule traversée par un débit (25).

**Fig. 1**

**Fig. 2**

EP 1 076 823 B1

**Fig. 3**

**Fig. 4**

12

**Fig. 5**